# EUROPEAN PATENT APPLICATION

(11) **EP 3 788 953 A1**
(43) Date of publication of application: **10.03.2021**
(21) Application number: 19796691.4
(22) Date of filing: 01.04.2019
(51) Int. Cl.: A61B 5/02

(54) **ELECTRONIC DEVICE**

(30) Priority: 01.05.2018 JP 2018088110
(71) Applicant: Kyocera Corporation, Kyoto-shi, Kyoto 612-8501 (JP)
(72) Inventor: AJIMA, Hiromi, Kyoto-shi, Kyoto 612-8501 (JP)
(74) Representative: Viering, Jentschura & Partner mbB Patent- und Rechtsanwälte
(86) International application number: PCT/JP2019/014471
(87) International publication number: WO 2019/211961

(57) **Abstract**

An electronic device comprises: an exterior portion including a holding surface configured to be held by a user; and a measurement portion displaceable with respect to the exterior portion, wherein the measurement portion includes: an arm displaceable according to a pulse wave of a user; and a sensor configured to detect displacement of the arm based on the pulse wave.

## Description

### CROSS REFERENCE TO RELATED APPLICATION

This application claims priority to Japanese Patent Application No. 2018-088110 filed on May 1, 2018, the entire disclosure of which is incorporated herein by reference.

### TECHNICAL FIELD

The present disclosure relates to an electronic device.

### BACKGROUND

An electronic device that acquires biological information of a subject in a state of being worn on the subject's wrist is conventionally known (for example, see PTL 1 and PTL 2).

### CITATION LIST

### Patent Literature

PTL 1: WO 2016/174839 A1
PTL 2: WO 2016/194308 A1

### SUMMARY

An electronic device according to an aspect comprises: an exterior portion including a holding surface configured to be held by a user; and a measurement portion displaceable with respect to the exterior portion. The measurement portion includes: an arm displaceable according to a pulse wave of a user; and a sensor configured to detect displacement of the arm based on the pulse wave.

### BRIEF DESCRIPTION OF THE DRAWINGS

In the accompanying drawings:
FIG. 1 is a schematic external perspective diagram of an electronic device according to an embodiment;
FIG. 2 is a schematic external perspective diagram of the electronic device according to the embodiment;
FIG. 3 is a schematic diagram illustrating a manner in which a subject measures biological information using the electronic device according to the embodiment;
FIG. 4 is a schematic external perspective diagram illustrating a non-use state of the electronic device according to the embodiment;
FIG. 5 is a schematic diagram illustrating a sensor and a body;
FIG. 6 is a schematic diagram illustrating the positional relationship between the subject's wrist and an arm when the electronic device is viewed from left;
FIG. 7 is a functional block diagram illustrating a schematic configuration of the electronic device according to the embodiment;
FIG. 8 is a schematic external perspective diagram illustrating an example of the electronic device including a notification interface;
FIG. 9 is a diagram illustrating an example of a pulse wave acquired by the sensor;

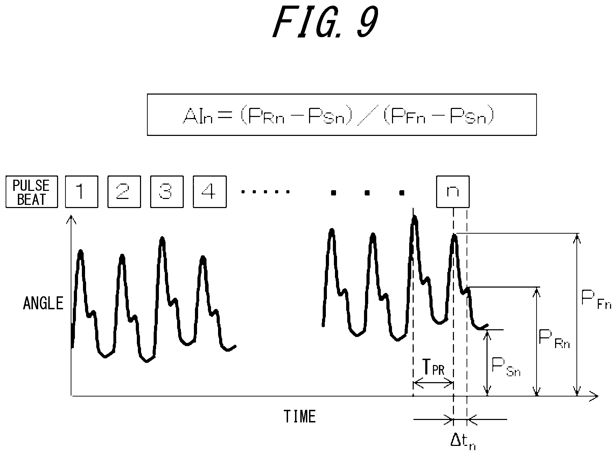

FIG. 9 is a diagram illustrating an example of a pulse wave acquired by the sensor;

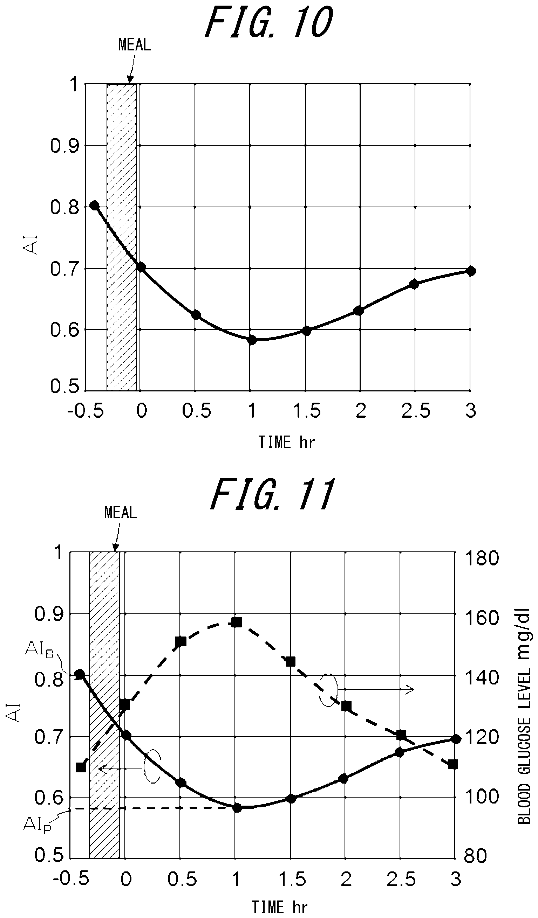

FIG. 10 is a diagram illustrating changes in a calculated AI over time;
FIG. 11 is a diagram illustrating the calculated AI and blood glucose level measurement results;

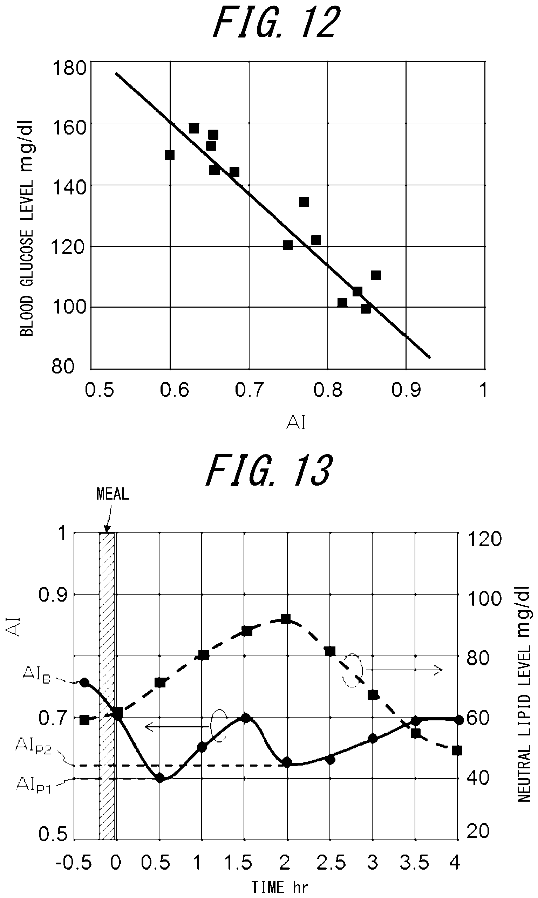

FIG. 12 is a diagram illustrating the relationship between the calculated AI and the blood glucose level;
FIG. 13 is a diagram illustrating the calculated AI and neutral lipid level measurement results;

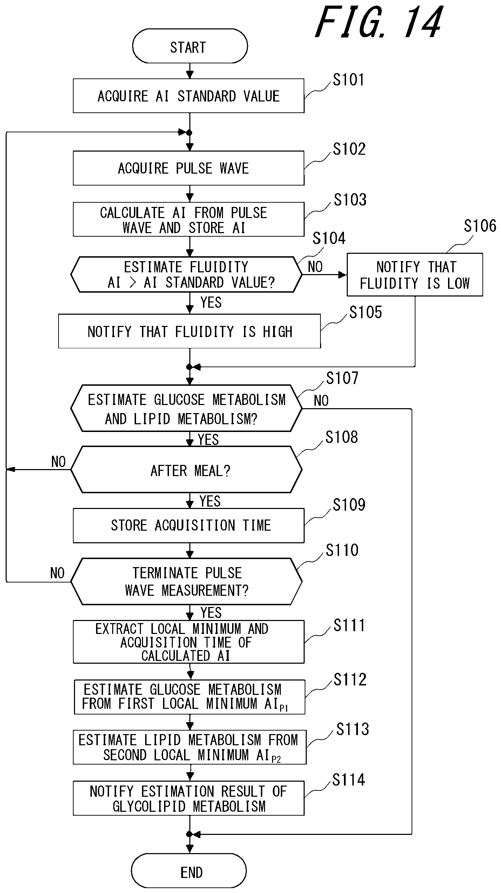

FIG. 14 is a flowchart illustrating a procedure to estimate blood fluidity, glucose metabolism, and lipid metabolism;

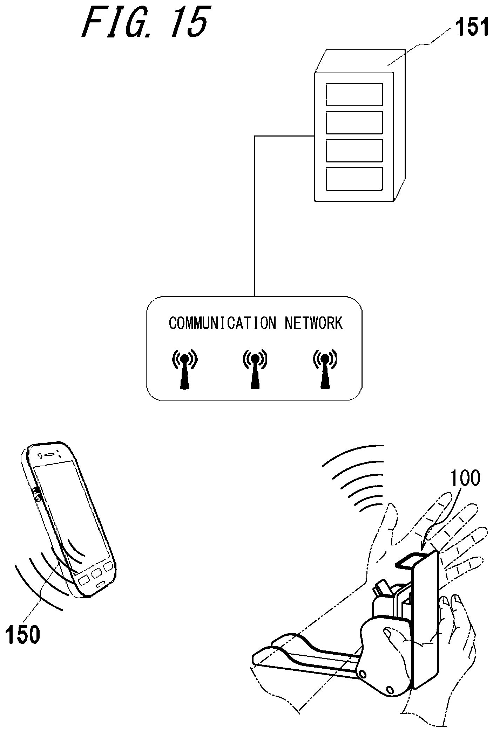

FIG. 15 is a schematic diagram illustrating a configuration of a system according to the embodiment;

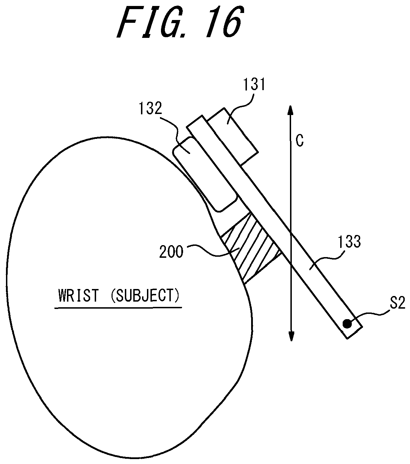

FIG. 16 is a schematic diagram illustrating the sensor and the arm;

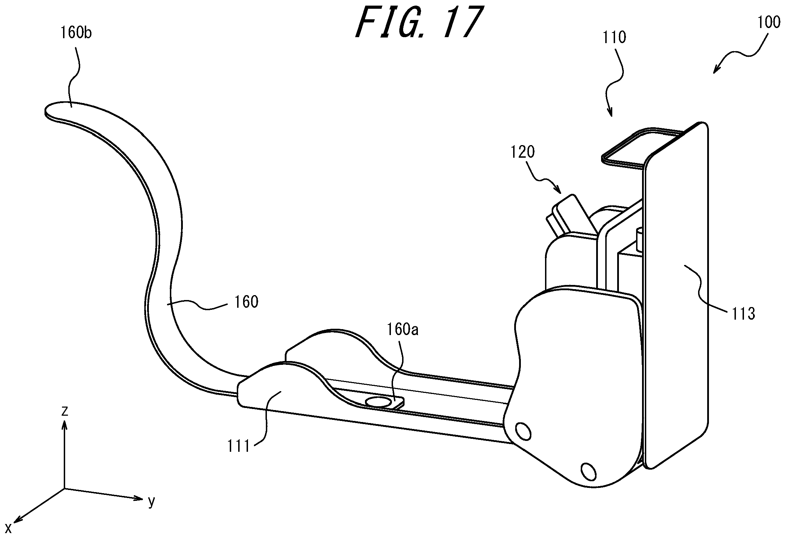

FIG. 17 is a schematic external perspective diagram of a modification of the electronic device;

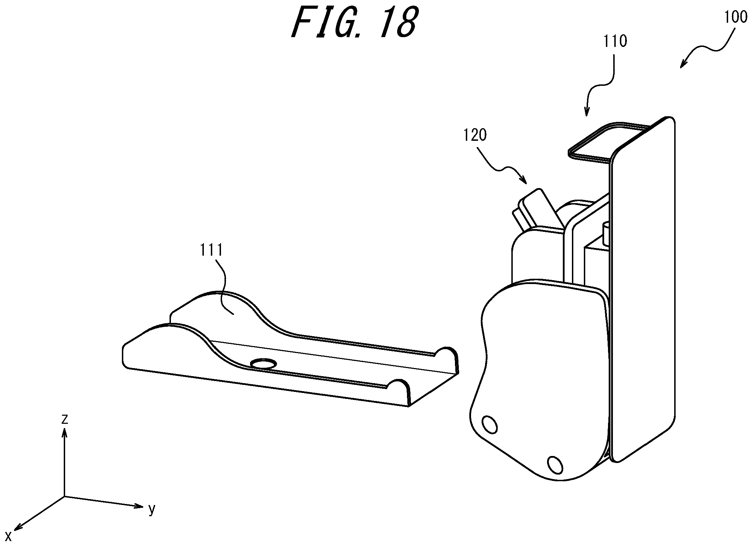

FIG. 18 is a schematic external perspective diagram of a modification of the electronic device;

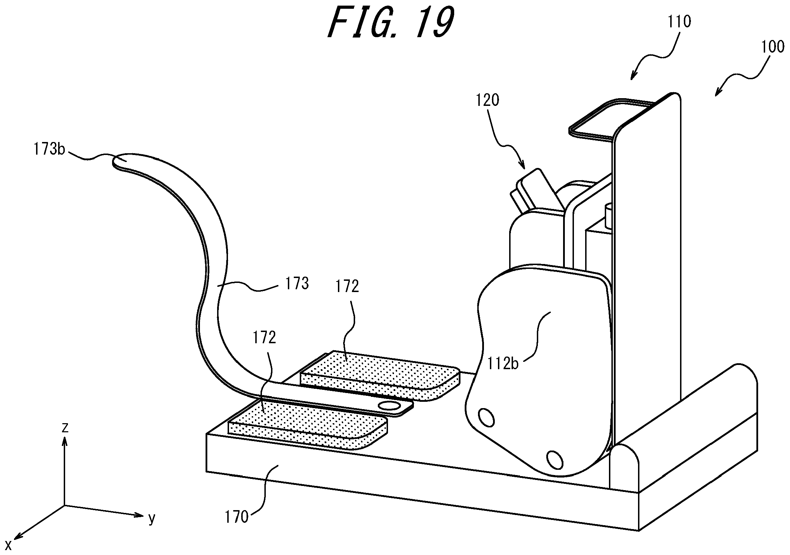

FIG. 19 is an external perspective diagram illustrating an example of a state in which the electronic device is mounted on a base; and

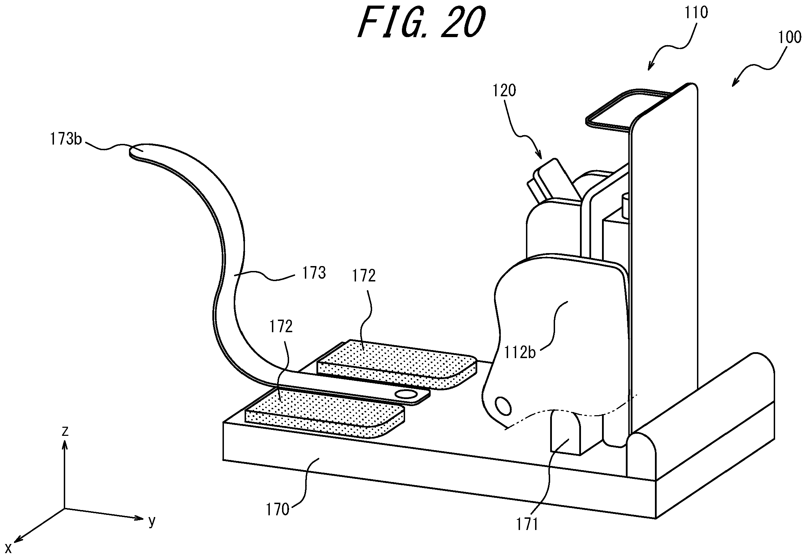

FIG. 20 is a diagram illustrating an example of a state in which the electronic device and the base are joined together.

### DETAILED DESCRIPTION

Biological information cannot be easily measured depending on the configuration of an electronic device. An electronic device configured to acquire biological information more easily is more useful to a subject. It could therefore be helpful to provide an electronic device having enhanced usefulness. According to the present disclosure, an electronic device having enhanced usefulness can be provided. Embodiments will be described in detail below, with reference to the drawings.

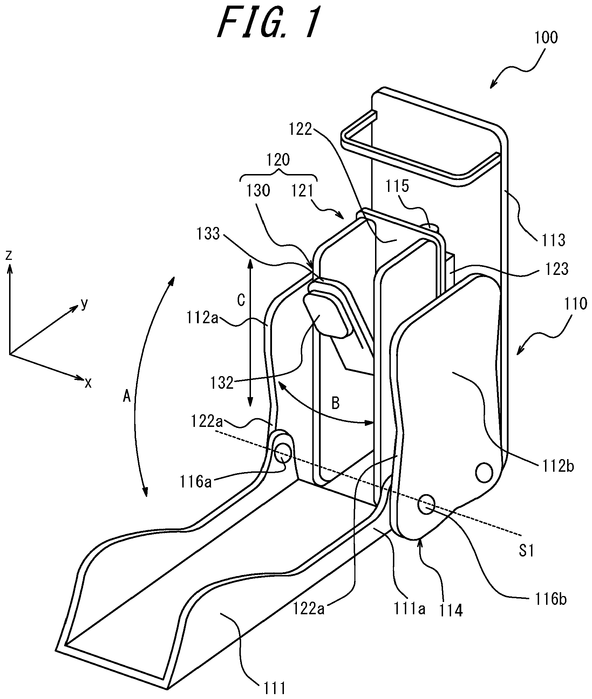

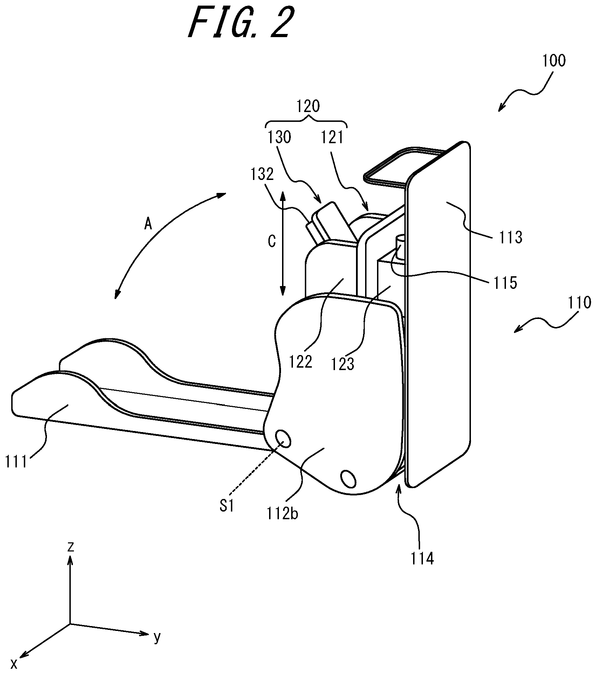

FIGS. 1 and 2 are each a schematic external perspective diagram of an electronic device 100 according to an embodiment. FIGS. 1 and 2 schematically illustrate the electronic device 100 as viewed from different points of view. The electronic device 100 includes an exterior portion 110 and a measurement portion 120.

The electronic device 100 measures biological information of a subject in a state in which a measured part of the subject is in contact with the measurement portion 120. The biological information measured by the electronic device 100 is the subject's pulse wave that is measurable by the measurement portion 120. This embodiment describes an example in which the electronic device 100 acquires the pulse wave with the subject's wrist being in contact with the measurement portion 120.

The measurement portion 120 is used to measure the biological information. The exterior portion 110 covers at least part of the measurement portion 120 from outside. The exterior portion 110 can protect the covered part of the measurement portion 120. When measuring the biological information using the electronic device 100, the subject holds the exterior portion 110 with one hand to support the electronic device 100.

The exterior portion 110 includes a cover 111, two side portions 112a and 112b, a back portion 113, and a bottom portion 114. In the exterior portion 110, the two side portions 112a and 112b and the back portion 113 form a holding surface held by the subject when measuring the biological information.

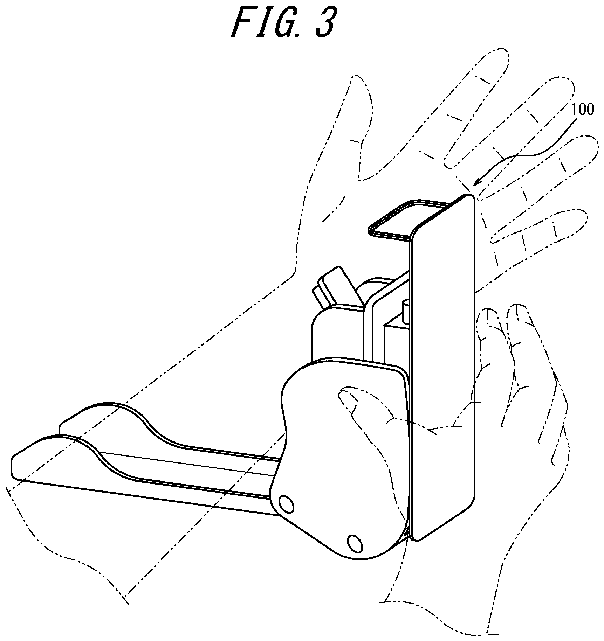

FIG. 3 is a schematic diagram illustrating a manner in which the subject measures the biological information using the electronic device 100. In the case of measuring the biological information, the subject places the electronic device 100 on a stand such as a desk so that the cover 111 and the bottom portion 114 will be in contact with the stand. At least part of the cover 111 and/or the bottom portion 114 in contact with the stand may be provided with a slip resistance such as rubber. The provision of the slip resistance eases stable placement of the electronic device 100 on the stand.

In a state in which the electronic device 100 is placed on the stand, the subject places the wrist on the cover 111 and presses the wrist against the measurement portion 120. For example, the subject presses the wrist against the measurement portion 120 so that the below-described pulse pad 132 of the measurement portion 120 will be in contact with a part where the ulnar artery or the radial artery is present. Here, by supporting the holding surface with the other hand whose wrist is not pressed against the pulse pad 132 and pressing the holding surface against the wrist, the subject can maintain the contact state between the pulse pad 132 and the wrist. The electronic device 100 measures the pulse wave of blood flowing through the ulnar artery or the radial artery at the subject's wrist.

With reference to FIGS. 1 and 2, in this embodiment, the back portion 113 has a substantially rectangular flat plate shape. Herein, the short-side direction of the back portion 113 in a substantially rectangular flat plate shape is referred to as "x-axis direction", the long-side direction of the back portion 113 in a substantially rectangular flat plate shape is referred to as "z-axis direction", and a direction orthogonal to the back portion 113 in a flat plate shape (i.e. a direction orthogonal to the xz plane) is referred to as "y-axis direction", as illustrated in FIGS. 1 and 2. Part of the electronic device 100 is movable, as described herein. The directions described herein with regard to the electronic device 100 correspond to the x-, y-, and z-axis directions in the state illustrated in FIGS. 1 and 2, unless stated otherwise. Herein, the z-axis positive direction is upward, and the z-axis negative direction is downward. The y-axis negative direction is toward the front side of the electronic device 100, and the y-axis positive direction is toward the back side of the electronic device 100. The x-axis positive direction is toward the left side of the electronic device 100, and the x-axis negative direction is toward the right side of the electronic device 100.

The bottom portion 114 has, for example, a flat plate shape. In the electronic device 100, the bottom portion 114 intersects at right angles with the back portion 113 at the lower short side of the substantially rectangular back portion 113. The back portion 113 and the bottom portion 114 may be fixed to each other. A shaft 115 extending along the holding surface, i.e. extending in the z-axis direction, is fixed to the bottom portion 114.

The two side portions 112a and 112b each have a flat plate shape. In the electronic device 100, the two side portions 112a and 112b intersect at right angles with the back portion 113 at the respective two long sides of the substantially rectangular back portion 113. The back portion 113 and each of the two side portions 112a and 112b may be fixed to each other.

In this embodiment, the side portions 112a and 112b and the back portion 113 forming the holding surface in the exterior portion 110 are U-shaped as viewed from above. The holding surface extends in the z-axis direction.

In the electronic device 100, the back portion 113 protects the back side of the measurement portion 120, the bottom portion 114 protects the bottom side of the measurement portion 120, and the two side portions 112a and 112b protect the right and left sides of the measurement portion 120.

The cover 111 includes a member having a substantially rectangular flat plate shape, and members extending from the long sides of the flat plate-shaped member so as to be orthogonal to the flat plate-shaped member. The subject places the wrist on the members orthogonal to the flat plate-shaped member in the cover 111, and measures the biological information by the electronic device 100, as illustrated in FIG. 3 as an example. By placing the wrist on the cover 111 during the measurement of the biological information, the subject can stabilize the position of the wrist. Consequently, the subject can bring the wrist into contact with the measurement portion 120 stably. This contributes to higher biological information measurement accuracy.

The cover 111 is connected to the side portions 112a and 112b at one end 111a. The end 111a is the back end in the state illustrated in FIG. 1. The cover 111 is connected to the side portions 112a and 112b so as to be rotatable in the yz plane about a straight line (axis) S1 linking connections 116a and 116b between the cover 111 and the respective side portions 112a and 112b, as indicated by arrow A in FIG. 1. That is, the cover 111 can be displaced between two states, i.e. a state in which the flat plate-shaped member lies along the xy plane as illustrated in FIG. 1 and a state in which the flat plate-shaped member lies along the xz plane as a result of rotation in the yz plane about the axis S1. The state in which the flat plate-shaped member of the cover 111 lies along the xy plane as illustrated in FIG. 1 is hereafter also referred to as a "state in which the electronic device 100 is open". The state in which the flat plate-shaped member of the cover 111 lies along the xz plane is hereafter also referred to as a "state in which the electronic device 100 is closed".

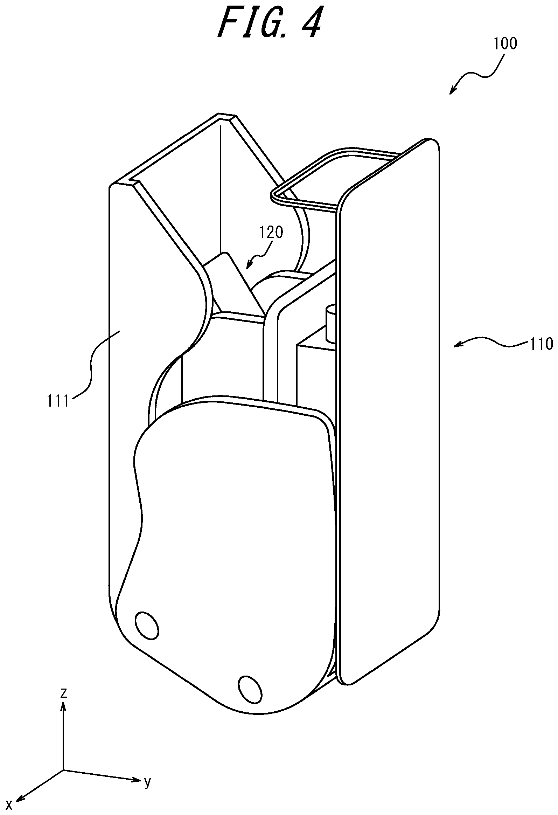

FIG. 4 is a schematic external perspective diagram illustrating a non-use state of the electronic device 100, i.e. a state in which the electronic device 100 is not used to measure the biological information. When the electronic device 100 is not in use, the subject can close the cover 111 of the electronic device 100 as illustrated in FIG. 4. As a result of closing the cover 111, the front side of the measurement portion 120 is protected. Moreover, as a result of closing the cover 111, the electronic device 100 is in a state of being folded up in a smaller size than in the open state, so that the subject can easily carry the electronic device 100 in a case, a bag, or the like.

With reference to FIGS. 1 and 2, the measurement portion 120 includes a body 121 and a sensor 130.

The body 121 includes a wall portion 122 having walls in three directions of the right, left, and back sides. That is, the wall portion 122 is U-shaped when the electronic device 100 is viewed from above.

The body 121 includes a connection 123 on the back side of the wall portion 122. The connection 123 has a bearing through which the shaft 115 passes. As a result of the shaft 115 being inserted through the bearing, the measurement portion 120 is attached to the exterior portion 110 via the shaft 115. Thus, the measurement portion 120 is attached to the exterior portion 110 so as to be rotatable about the shaft 115 in the xy plane intersecting the side portions 112a and 112b and the back portion 113 constituting the holding surface, as indicated by arrow B in FIG. 1. Thus, the measurement portion 120 is attached to the exterior portion 110 so as to be rotatable along the xy plane with respect to the exterior portion 110.

The measurement portion 120 is also attached to the exterior portion 110 so as to be vertically displaceable with respect to the exterior portion 110 along the shaft 115, i.e. in the z-axis direction, as indicated by arrow C in FIGS. 1 and 2. In this embodiment, the shaft 115 extends along the holding surface, so that the measurement portion 120 is displaceable along the holding surface.

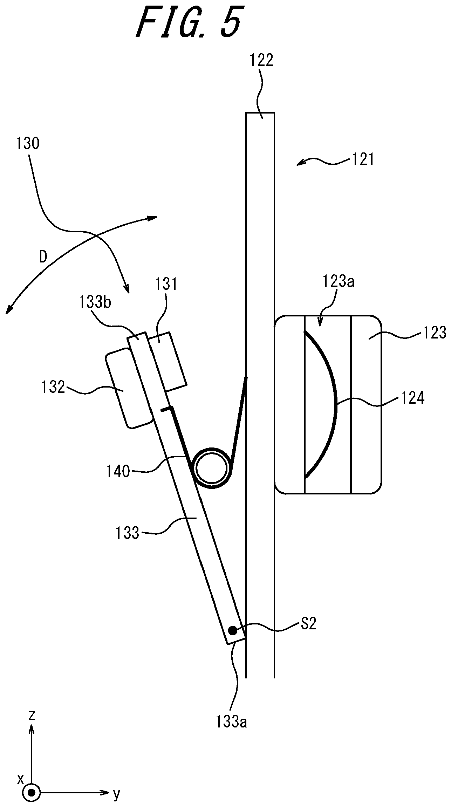

The sensor 130 is located in a space surrounded by the walls in the wall portion 122 of the body 121. The measurement portion 120 will be described in detail below, with reference to FIG. 5. FIG. 5 is a schematic diagram illustrating the sensor 130 and the body 121. FIG. 5 is a sectional diagram of a center part of the electronic device 100 in a front view taken along the yz plane, and illustrates the sensor 130 and the body 121 when the electronic device 100 is viewed from left. For the wall portion 122, only the back wall is illustrated in FIG. 5.

As illustrated in FIG. 5, the body 121 includes the connection 123 on the back side of the wall portion 122. The connection 123 has a bearing 123a through which the shaft 115 passes. A plate spring 124 is located inside the bearing 123a. By the elastic force of the plate spring 124, the vertical position of the measurement portion 120 with respect to the shaft 115 is fixed at a predetermined position.

The sensor 130 includes an arm 133. The arm 133 is connected to the wall portion 122 at one end 133a. For example, the arm 133 has a bearing at the end 133a, and is connected to the wall portion 122 as a result of a shaft S2 connected to the right and left surfaces of the wall portion 122 being inserted through the bearing. As a result of the sensor 130 being connected to the wall portion 122 of the body 121 in this way, the arm 133 is, at the other end 133b, rotatable in the yz plane about the shaft S2 as an axis, as indicated by arrow D in FIG. 5. It suffices for the arm 133 to be displaceable along a plane intersecting with the plane in which the measurement portion 120 is rotationally displaced.

The arm 133 is connected to the front wall surface of the wall portion 122 via an elastic body 140. The arm 133 is connected to the wall portion 122 so that, in a state in which the elastic body 140 is not pressed, the other end 133b will be located forward from an end 122a of the wall portion 122 on the front side. In detail, in a state in which the elastic body 140 is not pressed, the other end 133b is located forward from the wall portion 122 when the measurement portion 120 is viewed from the left side of the electronic device 100.

The elastic body 140 may be, for example, a spring. The elastic body 140 is not limited to a spring, however, and can be any other elastic body such as a resin or a sponge. In the example illustrated in FIG. 5, the elastic body 140 is a torsion coil spring, and connects a center part of the arm 133 between the one end 133a and the other end 133b to the wall portion 122.

The pulse pad 132 is joined to the other end 133b of the arm 133. The pulse pad 132 is a part in contact with a measured part of the subject subjected to blood pulse wave measurement when measuring the biological information using the electronic device 100. In this embodiment, for example, the subject brings a part where the ulnar artery or the radial artery is present into contact with the pulse pad 132. The pulse pad 132 may be made of a material that does not easily absorb changes in the body surface due to the pulse of the subject. The pulse pad 132 may be made of a material not painful to the subject in a contact state. For example, the pulse pad 132 may be made of a fabric bag filled with beads. The pulse pad 132 may be, for example, removable from the arm 133. For example, the subject may mount, on the arm 133, one of a plurality of pulse pads 132 different in size and/or shape, depending on the size and/or shape of the wrist. The subject can thus use the pulse pad 132 suitable for the size and/or shape of the wrist.

The sensor 130 includes an angular velocity sensor 131, such as a gyro sensor, that detects displacement of the arm 133. It suffices for the angular velocity sensor 131 to be capable of detecting angular displacement of the arm 133. The type of sensor provided in the sensor 130 is not limited to the angular velocity sensor 131, and may be, for example, an acceleration sensor, an angle sensor, any other motion sensor, or a combination thereof.

In this embodiment, in the measurement of the biological information by the electronic device 100, the subject brings the pulse pad 132 into contact with the skin above the radial artery which is the artery on the thumb side of the subject's left hand, as illustrated in FIG. 3 as an example. By the elastic force of the elastic body 140 located between the wall portion 122 and the arm 133, the pulse pad 132 at the other end 133b of the arm 133 is in contact with the skin above the subject's radial artery. The arm 133 is displaced according to the movement of the subject's radial artery, i.e. pulsation. The angular velocity sensor 131 acquires the pulse wave by detecting the displacement of the arm 133. The pulse wave refers to a waveform representation of the temporal change in volume of a blood vessel due to inflow of blood, acquired from the body surface.

In a state in which the elastic body 140 is not pressed, the other end 133b of the arm 133 is located forward from the front end of the wall portion 122, as mentioned above. When the subject brings the pulse pad 132 into contact with the skin above the radial artery, the elastic body 140 expands and contracts according to pulsation, and the pulse pad 132 is displaced. A component with an appropriate elastic modulus is used for the elastic body 140 so as to allow it to expand and contract according to pulsation without inhibiting pulsation.

The electronic device 100 may include various functional units used for pulse wave measurement by the electronic device 100, at appropriate positions in the exterior portion 110 or the measurement portion 120. For example, the electronic device 100 may include the below-described controller, power source, memory, communication interface, notification interface, circuitry for causing these components to operate, cables for connecting these components, and the like.

The movement of the movable portions in the electronic device 100 in the case of measuring the biological information using the electronic device 100 will be described below.

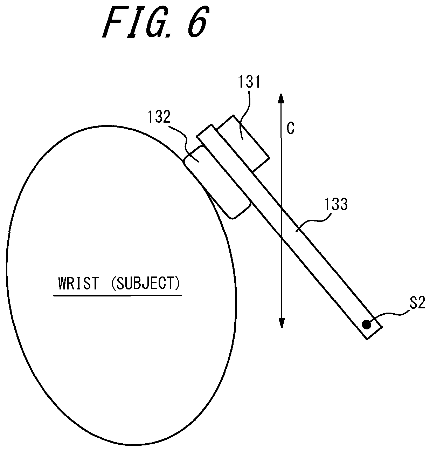

In the case of measuring the biological information using the electronic device 100, in a state in which the electronic device 100 is placed on the stand, the subject puts the wrist on the cover 111, and presses the wrist against the measurement portion 120, as mentioned above. The subject then brings the pulse pad 132 into contact with the skin above the radial artery at the wrist. Since the measurement portion 120 is displaceable in the direction of arrow C in FIG. 1, the arm 133 of the sensor 130 in the measurement portion 120 is equally displaceable in the direction of arrow C, i.e. the z-axis direction, as illustrated in FIG. 6. Hence, the subject can displace the arm 133 in the direction of arrow C depending on the size, thickness, etc. of the wrist so that the pulse pad 132 will be in contact with the skin above the radial artery. At the displacement position, the subject can fix the vertical position of the measurement portion 120 by the plate spring 124. Thus, with the electronic device 100, the position of the sensor 130 can be easily adjusted to a position suitable for measurement. The measurement accuracy of the electronic device 100 is therefore improved. Although the measurement portion 120 is displaceable in the z-axis direction in the example illustrated in FIG. 1, the measurement portion 120 need not necessarily be displaceable in the z-axis direction. It suffices for the measurement portion 120 to be positionally adjustable depending on, for example, the size and thickness of the wrist. For example, the measurement portion 120 may be displaceable in a direction intersecting with the xy plane.

In the case where the pulse pad 132 is in contact with the skin above the radial artery in a direction orthogonal to the skin surface, the pulsation transmitted to the arm 133 increases. In detail, in the case where the displacement direction of the pulse pad 132 (direction of arrow D in FIG. 5) is orthogonal to the skin surface, the pulsation transmitted to the arm 133 increases, with it being possible to improve the pulsation acquisition accuracy. In the electronic device 100 according to this embodiment, the measurement portion 120 is rotatable about the shaft 115 with respect to the exterior portion 110, as indicated by arrow B in FIG. 1. The subject can accordingly adjust the direction of the measurement portion 120 so that the displacement direction of the pulse pad 132 will be orthogonal to the skin surface. That is, in the electronic device 100, the direction of the measurement portion 120 can be adjusted so that the displacement direction of the pulse pad 132 will be orthogonal to the skin surface. Thus, with the electronic device 100, the direction of the measurement portion 120 can be adjusted depending on the shape of the subject's wrist. This facilitates transmission of the change in the pulsation of the subject to the arm 133. The measurement accuracy of the electronic device 100 is therefore improved.

Although the measurement portion 120 is rotatable along the xy plane in the example illustrated in FIG. 1, the measurement portion 120 need not necessarily be displaceable along the xy plane. For example, it suffices for the measurement portion 120 to be configured so that the contact direction of the pulse pad 132 with respect to the skin surface is adjustable. For example, the measurement portion 120 may be displaceable along a plane intersecting with the holding surface.

After bringing the pulse pad 132 into contact with the skin above the radial artery at the wrist, the subject holds the exterior portion 110 with the hand not in contact with the pulse pad 132. In the example illustrated in FIG. 3, the subject brings the pulse pad 132 into contact with the skin above the radial artery at the wrist of the left hand, and holds the exterior portion 110 with the right hand. The subject may rotate the exterior portion 110 held with the right hand about the axis S1 as an axis in a counterclockwise direction when the electronic device 100 is viewed from left, to displace the upper end of the back portion 113 in the y-axis negative direction. Consequently, the body 121 is displaced in the y-axis negative direction, too. As a result of the body 121 being displaced in the y-axis negative direction, the pulse pad 132 is biased toward the radial artery side by the elastic force of the elastic body 140. This allows the pulse pad 132 to capture the change in pulsation more reliably. The measurement accuracy of the electronic device 100 is therefore improved.

The rotation direction of the back portion 113 (direction of arrow A) and the rotation direction of the arm 133 (direction of arrow D) may be substantially parallel. If the rotation direction of the back portion 113 and the rotation direction of the arm 133 are closer to parallel, the elastic force of the elastic body 140 is applied to the arm 133 more efficiently when the upper end of the back portion 113 is displaced in the y-axis negative direction. The range in which the rotation direction of the back portion 113 and the rotation direction of the arm 133 are substantially parallel includes the range in which the elastic force of the elastic body 140 is applied to the arm 133 when the upper end of the back portion 113 is displaced in the y-axis negative direction.

When the upper end of the back portion 113 is displaced in the y-axis negative direction, the end 122a of the wall portion 122 on the front side comes into contact with the wrist. As a result of the end 122a coming into contact with the wrist, the back portion 113 is kept from being displaced in the y-axis negative direction beyond the contact position. Thus, the end 122a can prevent the back portion 113 from being displaced beyond the predetermined position. If the back portion 113 is displaced in the y-axis negative direction beyond the predetermined position, the arm 133 is strongly biased toward the radial artery side by the elastic force of the elastic body 140. This is likely to hinder the pulsation of the radial artery. In the electronic device 100 according to this embodiment, on the other hand, the wall portion 122 has the end 122a, so that the radial artery can be kept from being subjected to excessive pressure from the arm 133. The pulsation of the radial artery is therefore unlikely to be hindered. The end 122a thus functions as a stopper that limits the displaceable range of the back portion 113.

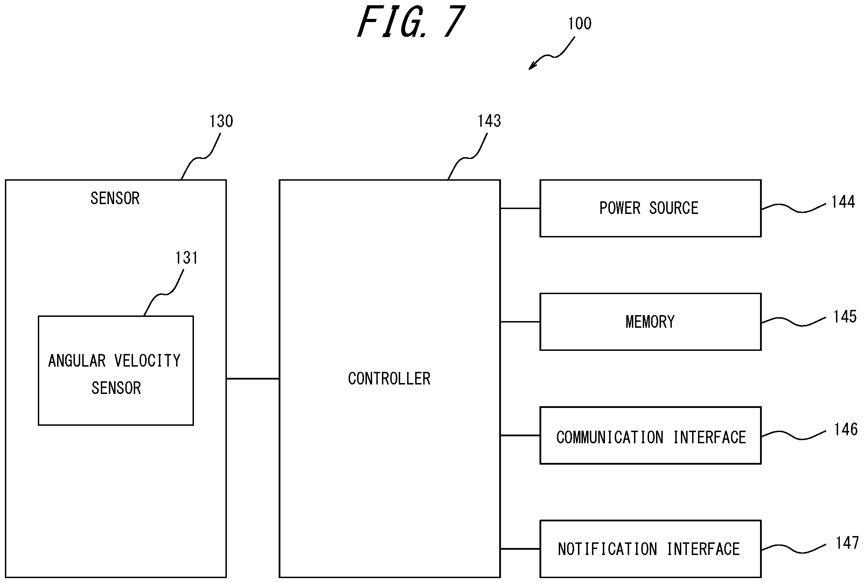

FIG. 7 is a functional block diagram illustrating the configuration of the electronic device 100. The electronic device 100 includes the sensor 130, a controller 143, a power source 144, a memory 145, a communication interface 146, and a notification interface 147. In this embodiment, the controller 143, the power source 144, the memory 145, the communication interface 146, and the notification interface 147 are included, for example, inside the exterior portion 110.

The sensor 130 includes the angular velocity sensor 131, detects pulsation from the measured part, and acquires the pulse wave.

The controller 143 is a processor for overall control and management of the electronic device 100, including, for example, the functional blocks of the electronic device 100. Furthermore, the controller 143 is a processor that calculates an index based on a pulse wave propagation phenomenon using the acquired pulse wave. The controller 143 is configured using a processor such as a central processing unit (CPU) that executes a program prescribing control procedures and a program that calculates the index based on the pulse wave propagation phenomenon. These programs are, for example, stored in a storage medium such as the memory 145. According to the calculated index, the controller 143 estimates a state related to the subject's glucose metabolism, lipid metabolism, or the like.

The power source 144 includes, for example, a lithium-ion battery and a control circuit for charging and discharging the lithium-ion battery. The power source 144 supplies power to the electronic device 100 overall.

The memory 145 stores programs and data. The memory 145 may include any non-transitory storage medium, such as a semiconductor storage medium and a magnetic storage medium. The memory 145 may also include a plurality of types of storage media. The memory 145 may include a combination of a portable storage medium, such as a memory card, an optical disc, or a magneto-optical disc, and an apparatus for reading the storage medium. The memory 145 may include a storage device used as a temporary storage area, such as random access memory (RAM). The memory 145 stores a variety of information, programs for causing the electronic device 100 to operate, and the like, and also functions as a working memory. The memory 145 may, for example, store the measurement result of the pulse wave acquired by the sensor 130.

The communication interface 146 exchanges a variety of data with an external apparatus by wired or wireless communication. For example, the communication interface 146 communicates with an external apparatus that stores the biological information of the subject to manage the state of health. The communication interface 146 transmits the measurement result of the pulse wave measured by the electronic device 100 and the state of health estimated by the electronic device 100 to the external apparatus.

The notification interface 147 provides notification of information by sound, vibration, images, and the like. The notification interface 147 may include a speaker, a vibrator, and/or a display device such as a liquid crystal display (LCD), an organic electro-luminescence display (OELD), or an inorganic electro-luminescence display (IELD). In this embodiment, for example, the notification interface 147 provides notification of the measurement result of the biological information such as the state of the subject's glucose metabolism or lipid metabolism.

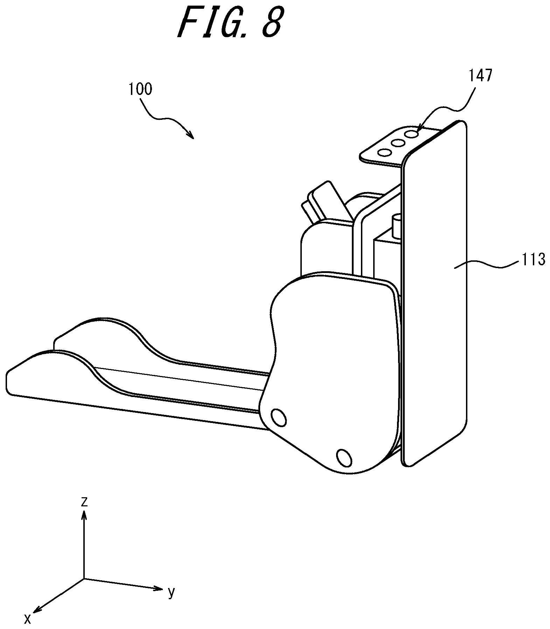

FIG. 8 is a schematic external perspective diagram illustrating an example of the electronic device 100 including the notification interface 147. In the example illustrated in FIG. 8, the notification interface 147 is composed of three light emitting diodes (LEDs). The three LEDs are provided at the upper end of the back portion 113. The three LEDs may emit light in different colors. In the example illustrated in FIG. 8, the controller 143 may cause the LEDs to illuminate in a color and/or pattern different depending on the measurement result of the biological information. The subject can then recognize the measurement result of the biological information from the emission color and/or pattern of the LEDs.

FIG. 9 is a diagram illustrating an example of a pulse wave acquired at the wrist using the electronic device 100. FIG. 9 illustrates the case where the angular velocity sensor 131 is used as a means for detecting the pulsation. FIG. 9 illustrates a time integration of the angular velocity acquired by the angular velocity sensor 131, with the horizontal axis representing time and the vertical axis representing the angle. Since the acquired pulse wave may, for example, include noise caused by body movement of the subject, the pulse wave may be corrected by a filter that removes the direct current (DC) component, so as to extract only the pulsation component.

A method of calculating a pulse wave index from the acquired pulse wave will be described below, with reference to FIG. 9. Propagation of the pulse wave is a phenomenon in which pulsation due to blood being pumped from the heart is transmitted through artery walls and blood. The pulsation due to blood pumped from the heart reaches the peripheries of limbs as a forward wave, a portion of which is reflected at locations such as where a blood vessel branches, or where the diameter of a blood vessel changes, and returns as a reflected wave. The pulse wave index is, for example, the pulse wave velocity (PWV) of the forward wave, the magnitude P_{R} of the reflected wave of the pulse wave, the time difference Δt between the forward wave and the reflected wave of the pulse wave, or the augmentation index (AI) represented as the ratio between the magnitudes of the forward wave and the reflected wave of the pulse wave.

The pulse wave illustrated in FIG. 9 represents n pulse beats of the user, where n is an integer that is greater than or equal to 1. The pulse wave is a combined wave in which the forward wave generated by ejection of blood from the heart overlaps with the reflected wave generated at blood vessel branches and locations of change in blood vessel diameter. In FIG. 9, the magnitude of the peak in the pulse wave from the forward wave in each pulse beat is labeled P_{Fn}, the magnitude of the peak in the pulse wave from the reflected wave in each pulse beat is labeled P_{Rn}, and the smallest value of the pulse wave in each pulse beat is labeled Psn. In FIG. 9, the interval between peaks of the pulse wave is labeled T_{PR}.

The pulse wave index quantifies information obtained from the pulse wave. An example of a pulse wave index is PWV, which is calculated according to the difference in propagation time between pulse waves measured at two points, such as the upper arm and ankle, and the distance between the two points. Specifically, PWV is calculated by synchronously acquiring the pulse waves at two points on an artery (e.g. the upper arm and ankle) and dividing the distance between the two points (L) by the time difference of the pulse waves at the two points (PTT). Another example of a pulse wave index is the reflected wave magnitude P_{R}, which may be calculated as the magnitude P_{Rn} of the peak in the pulse wave from the reflected wave or as the average of n values, P_{Rave}. Another example of a pulse wave index is the time difference Δt between the forward wave and the reflected wave of the pulse wave, which may be calculated as the time difference Δtₙ between predetermined pulse beats or as the average of n time differences, Δtₐᵥₑ. Another example of a pulse wave index is the AI, which is the result of dividing the magnitude of the reflected wave by the magnitude of the forward wave and is represented as AIₙ = (P_{Rn} - P_{Sn})/(P_{Fn} - P_{Sn}). AIn is the AI for each pulse beat. As the pulse wave index, AI may, for example, be calculated by measuring the pulse wave for several seconds and calculating the average Alave of the AIn for each pulse beat (n = an integer from 1 to n).

The PWV, the reflected wave magnitude P_{R}, the time difference Δt between the forward wave and the reflected wave, and the AI indices can be used to estimate the state of arteriosclerosis because they change in dependence on the hardness of the blood vessel walls. The PWV increases, for example, if the blood vessel walls are hard. The reflected wave magnitude P_{R}, for example, also increases if the blood vessel walls are hard. The time difference Δt between the forward wave and the reflected wave, for example, decreases if the blood vessel walls are hard. The AI, for example, increases if the blood vessel walls are hard. Furthermore, by using these indices which are based on the pulse wave, the electronic device 100 can estimate the state of arteriosclerosis and also estimate the fluidity (viscosity) of blood. In particular, the electronic device 100 can estimate the change in blood fluidity from the change in indices based on pulse waves acquired for the same measured part of the same subject during a time period (such as several days) over which the state of arteriosclerosis exhibits essentially no change. Here, the "blood fluidity" indicates the ease of blood flow. The PWV, for example, decreases if the blood fluidity is low. The reflected wave magnitude P_{R}, for example, also decreases if the blood fluidity is low. The time difference Δt between the forward wave and the reflected wave, for example, increases if the blood fluidity is low. The AI, for example, decreases if the blood fluidity is low.

In this embodiment, as an example of pulse wave indices, the electronic device 100 calculates the PWV, the reflected wave magnitude P_{R}, the time difference Δt between the forward wave and the reflected wave, and the AI. However, the pulse wave indices are not limited to these examples. For example, the electronic device 100 may use posterior systolic blood pressure as a pulse wave index.

FIG. 10 illustrates the change over time in the calculated AI. In this embodiment, the pulse wave was acquired for approximately five seconds using the electronic device 100 including the angular velocity sensor 131. The controller 143 calculated the AI from the acquired pulse wave for each pulse beat and further calculated the average AIₐᵥₑ thereof. In this embodiment, the electronic device 100 acquired the pulse wave at a plurality of times before a meal and after the meal and calculated the average AI (simply "AI" below) as an example of an index based on the acquired pulse wave. The horizontal axis in FIG. 10 represents elapsed time, with the initial measurement time after a meal being 0. The vertical axis in FIG. 10 indicates the AI calculated from the pulse wave acquired at that time. The pulse wave is acquired on the radial artery, with the subject at rest.

The electronic device 100 acquired the pulse waves before a meal, immediately after the meal, and every 30 minutes after the meal, and calculated a plurality of AI values on the basis of the pulse waves. The AI calculated from the pulse wave acquired before the meal was approximately 0.8. The AI immediately after the meal was lower than before the meal, and the AI reached its lowest value approximately one hour after the meal. The AI gradually increased in three hours after the meal, until the completion of the measurement.

The electronic device 100 can estimate the change in blood fluidity from the change in the calculated AI. The blood fluidity decreases, for example, when red blood cells, white blood cells, and platelets in the blood harden into balls, or when the adhesive force increases. The blood fluidity also decreases, for example, when the moisture content of platelets in the blood decreases. These changes in the blood fluidity depend on the subject's state of health, such as the below-described glycolipid state, heatstroke, dehydration, hypothermia, and the like. Before the subject's state of health becomes critical, the subject can use the electronic device 100 according to this embodiment to learn about the subject's changes in blood fluidity. From the changes in AI before and after a meal as illustrated in FIG. 10, it can be inferred that the blood fluidity decreases after a meal, reaching a minimum approximately one hour after a meal and gradually increasing thereafter. The electronic device 100 may notify the user by expressing a low state of blood fluidity as "thick" and a high state of blood fluidity as "thin". For example, the electronic device 100 may make the determination of "thick" or "thin" taking the average AI for the subject's actual age as a standard. The electronic device 100 may determine the blood to be "thin" when the calculated AI is greater than the average and "thick" when the calculated AI is less than the average. The electronic device 100 may, for example, make the determination of "thick" or "thin" taking the preprandial AI as a standard. The electronic device 100 may compare the postprandial AI with the preprandial AI to estimate the degree of "thickness". The electronic device 100 can, for example, use the preprandial AI, i.e. the AI when fasting, as an index of the subject's vascular age (blood vessel hardness). For example, by calculating the amount of change in the AI calculated using the subject's preprandial AI, i.e. the AI when fasting, as a standard, the electronic device 100 can reduce the estimation error due to the subject's vascular age (blood vessel hardness). Hence, the change in blood fluidity can be estimated more accurately.

FIG. 11 is a diagram illustrating the calculated AI and the result of measuring the blood glucose level. The method of acquiring the pulse waves and the method of calculating AI are the same as in the embodiment illustrated in FIG. 10. The right vertical axis in FIG. 11 represents the blood glucose level, and the left vertical axis represents the calculated AI. The solid curve in FIG. 11 indicates the AI calculated from the acquired pulse waves, and the dotted curve indicates the measured blood glucose level. The blood glucose level was measured immediately after the pulse wave was acquired. The blood glucose level was measured using the blood glucose meter Medisafe Fit manufactured by Terumo Corporation. As compared with the preprandial blood glucose level, the postprandial blood glucose level rose by approximately 20 mg/dl. The blood glucose level reached the largest value approximately one hour after the meal. Subsequently, until measurement was completed, the blood glucose level decreased gradually and became nearly the same as the preprandial blood glucose level approximately three hours after the meal.

As illustrated in FIG. 11, the preprandial and postprandial blood glucose levels are negatively correlated with the AI calculated from the pulse wave. As the blood glucose level rises, the red blood cells and white blood cells harden into balls because of sugar in the blood, or the adhesive force increases. As a result, the blood fluidity decreases. Upon a decrease in the blood fluidity, the PWV may decrease. Upon a decrease in the PWV, the time difference Δt between the forward wave and the reflected wave may increase. Upon an increase in the time difference Δt between the forward wave and the reflected wave, the reflected wave magnitude P_{R} may decrease relative to the forward wave magnitude P_{F}. Upon a decrease in the reflected wave magnitude P_{R} relative to the forward wave magnitude P_{F}, the AI may decrease. Since the AI within the several hours following the meal (three hours in this embodiment) is correlated with the blood glucose level, variation in the subject's blood glucose level can be estimated from a change in AI. Furthermore, by measuring the subject's blood glucose level and acquiring the correlation with the AI in advance, the electronic device 100 can estimate the subject's blood glucose level from the calculated AI.

The electronic device 100 can estimate the state of the subject's glucose metabolism according to the occurrence time of AI_{P}, which is the first detected local minimum of the AI after a meal. For example, the electronic device 100 estimates the blood glucose level as the state of glucose metabolism. As an example of estimating the state of glucose metabolism, the electronic device 100 can infer that the subject has abnormal glucose metabolism (patient with diabetes) when the first detected local minimum AIₚ of the AI after a meal is detected after a predetermined length of time or longer (for example, approximately 1.5 hours or longer after a meal).

The electronic device 100 can estimate the state of the subject's glucose metabolism according to the difference AI_{B} - AI_{P} between AI_{B}, which is the preprandial AI, and AI_{P}, which is the first detected local minimum of the postprandial AI. As an example of estimating the state of glucose metabolism, the electronic device 100 can infer that the subject has abnormal glucose metabolism (patient with postprandial hyperglycemia) when AI_{B} - AI_{P} is greater than or equal to a predetermined value (for example, 0.5 or more).

FIG. 12 is a diagram illustrating the relationship between the calculated AI and the blood glucose level. The calculated AI and the blood glucose level were acquired within one hour after a meal, when the blood glucose level varies greatly. The data in FIG. 12 includes a plurality of different data points after a meal for the same subject. As illustrated in FIG. 12, the calculated AI and the blood glucose level are negatively correlated. The correlation coefficient between the calculated AI and the blood glucose level is 0.9 or higher, indicating an extremely high correlation. For example, by acquiring the correlation between the calculated AI and blood glucose level illustrated in FIG. 12 for each subject in advance, the electronic device 100 can estimate the subject's blood glucose level from the calculated AI.

FIG. 13 is a diagram illustrating the calculated AI and the result of measuring neutral lipids. The method of acquiring the pulse waves and the method of calculating AI are the same as in the embodiment illustrated in FIG. 10. The right vertical axis in FIG. 13 represents the neutral lipid level in the blood, and the left vertical axis represents the AI. The solid curve in FIG. 13 indicates the AI calculated from the acquired pulse waves, and the dotted curve indicates the measured neutral lipid level. The neutral lipid level was measured immediately after the pulse wave was acquired. The neutral lipid level was measured using the lipid measurement apparatus "Pocket Lipid" manufactured by Techno Medica Co., Ltd. As compared with the preprandial neutral lipid level, the highest value of the postprandial neutral lipid level represented a rise of approximately 30 mg/dl. The neutral lipid level reached the highest value approximately two hours after the meal. Subsequently, until measurement was completed, the neutral lipid level decreased gradually and became nearly the same as the preprandial neutral lipid level approximately three and a half hours after the meal.

By contrast, the local minimums of the calculated AI were a first local minimum AI_{P1} detected approximately 30 minutes after the meal and a second local minimum AI_{P2} detected approximately two hours after the meal. It can be inferred that the first local minimum AI_{P1} detected approximately 30 minutes after the meal is caused by the above-described blood glucose level after the meal. The occurrence time of the second local minimum AI_{P2}, which was detected approximately two hours after the meal, is nearly coincident with that of the highest neutral lipid level detected approximately two hours after the meal. From this, it can be inferred that the second local minimum AI_{P2} detected a predetermined length of time or longer after a meal is due to the effect of neutral lipids. Like the blood glucose level, it can be understood that the preprandial and postprandial neutral lipid values are negatively correlated with the AI calculated from the pulse wave. In particular, the local minimum AI_{P2} of the AI calculated a predetermined length of time or longer (in this embodiment, approximately 1.5 hours or longer) after a meal is correlated with neutral lipids. Therefore, the variation in the subject's neutral lipid level can be estimated from the variation in AI. Furthermore, by measuring the subject's neutral lipid level and acquiring the correlation with the AI in advance, the electronic device 100 can estimate the subject's neutral lipid level from the calculated AI.

The electronic device 100 can estimate the subject's state of lipid metabolism on the basis of the occurrence time of the second local minimum AI_{P2} detected a predetermined length of time or longer after a meal. For example, the electronic device 100 estimates the lipid level as the state of lipid metabolism. As an example of estimating the state of lipid metabolism, the electronic device 100 can infer that the subject has abnormal lipid metabolism (patient with hyperlipidemia) when the second local minimum AI_{P2} is detected a predetermined length of time or longer (for example, four hours or longer) after a meal.

The electronic device 100 can estimate the subject's state of lipid metabolism according to the difference AI_{B} - AI_{P2} between the AI_{B}, which is the preprandial AI, and the second local minimum AI_{P2} detected a predetermined length of time or longer after the meal. As an example, the electronic device 100 can infer that the subject's state of lipid metabolism is abnormal (patient with postprandial hyperlipidemia) when AI_{B} - AI_{P2} is 0.5 or more.

From the measurement results illustrated in FIGS. 11 to 13, the electronic device 100 according to this embodiment can estimate the subject's state of glucose metabolism according to the first local minimum AI_{P1}, detected earliest after a meal, and the occurrence time thereof. Furthermore, the electronic device 100 according to this embodiment can estimate the subject's state of lipid metabolism according to the second local minimum AI_{P2}, detected a predetermined length of time or longer after the first local minimum AI_{P1}, and the occurrence time thereof.

The case of neutral lipids has been described as an example of estimating the lipid metabolism in this embodiment, but estimation of the lipid metabolism is not limited to neutral lipids. The lipid level estimated by the electronic device 100 includes, for example, total cholesterol, high-density lipoprotein (HDL) cholesterol, and low-density lipoprotein (LDL) cholesterol. These lipid values also exhibit tendencies similar to the above-described case of neutral lipids.

FIG. 14 is a flowchart illustrating the procedure for estimating the blood fluidity and the state of glucose metabolism and lipid metabolism on the basis of the AI. The process by which the electronic device 100 according to this embodiment estimates the blood fluidity and the state of glucose metabolism and lipid metabolism will be described below, with reference to FIG. 14.

As illustrated in FIG. 14, the electronic device 100 acquires the subject's AI standard value as an initial setting (step S101). An average AI estimated from the subject's age or the subject's AI when fasting, acquired in advance, may be used as the AI standard value. The electronic device 100 may also use the AI determined to be before a meal in steps S102 to S108 or the AI calculated immediately before pulse wave measurement as the AI standard value. In this case, the electronic device 100 executes step S101 after steps S102 to S108.

Subsequently, the electronic device 100 acquires the pulse wave (step S102). For example, the electronic device 100 determines whether a pulse wave of predetermined amplitude or higher has been obtained during a predetermined measurement time (for example, five seconds). If a pulse wave of predetermined amplitude or higher has been obtained, the process proceeds to step S103. If a pulse wave of predetermined amplitude or higher has not been obtained, step S102 is repeated (these steps are not illustrated). In step S102, for example, upon detecting a pulse wave of predetermined amplitude or higher, the electronic device 100 automatically acquires the pulse wave.

From the pulse wave acquired in step S102, the electronic device 100 calculates the AI as a pulse wave index and stores the AI in the memory 145 (step S103). The electronic device 100 may calculate the average AIₐᵥₑ from the AIn (n = an integer from 1 to n) for each of a predetermined number of pulse beats (for example, three beats) and use the average AIₐᵥₑ as the AI. Alternatively, the electronic device 100 may calculate the AI for a specific pulse beat.

The AI may be calculated by correcting the AI in accordance, for example, with the pulse rate (PR), the pulse pressure (PF - PS), the body temperature, the temperature of the measured part, and the like. Pulse and AI are known to be negatively correlated, as are pulse pressure and AI. Temperature and AI are known to be positively correlated. When correcting the AI, for example, the electronic device 100 calculates the pulse and the pulse pressure in addition to the AI in step S103. For example, the electronic device 100 may include a temperature sensor in the sensor 130 and may acquire the temperature of the measured part when acquiring the pulse wave in step S102. The AI is corrected by substituting the acquired pulse, pulse pressure, temperature, and the like into a correction formula derived in advance.

Subsequently, the electronic device 100 compares the AI standard value acquired in step S101 and the AI calculated in step S103 and estimates the fluidity of the subject's blood (step S104). When the calculated AI is greater than the AI standard value (YES), then the electronic device 100 infers that the blood fluidity is high and for example provides a notification that "the blood is thin" (step S105). When the calculated AI is not greater than the AI standard value (NO), then the electronic device 100 infers that the blood fluidity is low and for example provides a notification that "the blood is thick" (step S106).

Subsequently, the electronic device 100 confirms with the subject whether to estimate the state of glucose metabolism and lipid metabolism (step S107). When it is confirmed in step S107 that the state of glucose metabolism and lipid metabolism is not to be estimated (NO), the electronic device 100 terminates the process. When it is confirmed in step 107 that the state of glucose metabolism and lipid metabolism is to be estimated (YES), the electronic device 100 confirms whether the calculated AI was acquired before a meal or after a meal (step S108). When acquisition was not after a meal, i.e. was before a meal (NO), the process returns to step S102, and the next pulse wave is acquired. When acquisition was after a meal (YES), the electronic device 100 stores the acquisition time of the pulse wave corresponding to the calculated AI (step S109). When continuing to acquire pulse waves (NO in step S110), the process returns to step S102, and the next pulse wave is acquired. When terminating pulse wave measurement (YES in step S110), the process proceeds to step Sill and beyond, and the electronic device 100 estimates the subject's state of glucose metabolism and lipid metabolism.

Subsequently, the electronic device 100 extracts the local minimums and the times thereof from a plurality of AI values calculated in step S104 (step S111). For example, in the case of the AI values illustrated by the solid curve in FIG. 13 being calculated, the electronic device 100 extracts the first local minimum AI_{P1} occurring 30 minutes after the meal and the second local minimum AI_{P2} occurring approximately two hours after the meal.

Subsequently, the electronic device 100 estimates the subject's state of glucose metabolism from the first local minimum AI_{P1} and the time thereof (step S112). Furthermore, the electronic device 100 estimates the subject's state of lipid metabolism from the second local minimum AI_{P2} and the time thereof (step S113). Examples of estimating the subject's state of glucose metabolism and lipid metabolism follow the examples described above in relation to FIGS. 11 to 13 and are therefore is omitted.

Subsequently, the electronic device 100 provides notification of the estimation result from step S112 and step S113 (step S114) and terminates the process illustrated in FIG. 14. For example, the notification interface 147 provides notifications such as "normal glucose metabolism", "suspected abnormal glucose metabolism", "normal lipid metabolism", or "suspected abnormal lipid metabolism". The notification interface 147 can also provide advice such as "seek advice from a doctor" or "improve your diet". The electronic device 100 then terminates the process illustrated in FIG. 14.

In this embodiment, the electronic device 100 can estimate the fluidity of the subject's blood and the state of glucose metabolism and lipid metabolism from an index based on the subject's pulse wave. Therefore, the electronic device 100 can estimate the fluidity of the subject's blood and the state of glucose metabolism and lipid metabolism in a non-invasive manner and in a short time.

In this embodiment, the electronic device 100 can estimate the state of glucose metabolism and estimate the state of lipid metabolism from the extreme values of indices based on the subject's pulse waves and the times thereof. Therefore, the electronic device 100 can estimate the subject's state of glucose metabolism and lipid metabolism in a non-invasive manner and in a short time.

In this embodiment, the electronic device 100 can, for example, estimate the subject's state of glucose metabolism and lipid metabolism using an index based on the subject's pulse wave before a meal (when fasting) as a standard. Therefore, the electronic device 100 can accurately estimate the fluidity of the subject's blood and the state of glucose metabolism and lipid metabolism without regard for the blood vessel diameter and blood vessel hardness, which do not exhibit short-term change.

In this embodiment, the electronic device 100 can estimate the subject's blood glucose level and lipid level in a non-invasive manner and in a short time by calibrating the index based on the subject's pulse wave with the blood glucose level and lipid level.

In this embodiment, when measuring the biological information using the electronic device 100, the subject can press the wrist of the hand having the measured part against the measurement portion 120, while supporting the exterior portion 110 and pressing the exterior portion 110 toward the wrist with the other hand. Thus, the subject can easily adjust the positional relationship between the measured part and the measurement portion 120 by moving the electronic device 100 with the other hand. Moreover, the subject can adjust the positional relationship between the measured part and the measurement portion 120 while visually checking it. Hence, with the electronic device 100, the measurement portion 120 can be appropriately and easily brought into contact with the measured part.

FIG. 15 is a schematic diagram illustrating a configuration of a system according to an embodiment. The system illustrated in FIG. 15 includes the electronic device 100, a server 151, a mobile terminal 150, and a communication network. As illustrated in FIG. 15, the following system can be established: The pulse wave index calculated by the electronic device 100 is transmitted to the server 151 through the communication network and stored in the server 151 as the subject's personal information. In the server 151, the fluidity of the subject's blood and the state of the subject's glucose metabolism and lipid metabolism are estimated by comparison with the subject's past acquired information and with a variety of databases. The server 151 further creates appropriate advice for the subject. The server 151 replies to the mobile terminal 150 in the subject's possession with estimation results and advice. The mobile terminal 150 provides notification, via a display of the mobile terminal 150, of the received estimation results and advice. By using the communication capability of the electronic device 100, information from a plurality of users can be collected in the server 151, thereby further improving the estimation accuracy. Moreover, since the mobile terminal 150 is used as a notification means, the electronic device 100 does not require the notification interface 147 and can be further reduced in size. Furthermore, since the fluidity of the subject's blood and the state of the subject's glucose metabolism and lipid metabolism are estimated by the server 151, the computation load on the controller 143 in the electronic device 100 can be reduced. In addition, the subject's past acquired information can be stored in the server 151, thereby reducing usage of the memory 145 in the electronic device 100. Therefore, the electronic device 100 can be further reduced in size and complexity. The processing speed for computation is also improved.

The system according to this embodiment is configured so that the electronic device 100 and the mobile terminal 150 are connected over the communication network via the server 151. However, the system according to the present disclosure is not limited to this configuration. The electronic device 100 and the mobile terminal 150 may be connected directly over the communication network without use of the server 151. For example, the electronic device 100 may transmit information to the mobile terminal 150 according to a protocol such as BLE (Bluetooth® Low Energy) (Bluetooth is a registered trademark in Japan, other countries, or both).

Characteristic embodiments have been described for a complete and clear disclosure. The appended claims, however, are not limited to the foregoing embodiments and are to be construed as encompassing all of the possible modifications and alternate configurations that a person of ordinary skill in the art could make within the scope of the fundamental features indicated in the present disclosure.

For example, although the foregoing embodiments describe the case where the sensor 130 includes the angular velocity sensor 131, the electronic device 100 is not limited to this. The sensor 130 may include an optical pulse wave sensor including an optical emitter and an optical detector, or may include a pressure sensor. The electronic device 100 is not limited to being worn on the wrist. It suffices for the sensor 130 to be placed on an artery, such as on the neck, ankle, thigh, or ear.

For example, although the foregoing embodiments describe the case of estimating preprandial and postprandial fluidity of blood, the electronic device 100 is not limited to this process. The electronic device 100 may estimate blood fluidity before, during, and after exercise, or estimate blood fluidity before, during, and after bathing.

In the foregoing embodiments, the natural frequency of the arm 133 may be close to the frequency of the pulse wave to be acquired. For example, in the case where the frequency of the pulse wave to be acquired is 0.5 Hz to 2 Hz (pulse rate: 30 to 120), the natural frequency of the arm 133 may be in a range of 0.5 Hz to 2 Hz. The natural frequency of the arm 133 can be optimized by changing the length or weight of the arm 133, the elastic modulus or spring constant of the elastic body 140, or the like. Such optimization of the natural frequency of the arm 133 enables the electronic device 100 to perform more accurate measurement.

Although the foregoing embodiments describe the case where the electronic device 100 measures the pulse wave, the pulse wave need not necessarily be measured by the electronic device 100. For example, the electronic device 100 may be connected to an information processing apparatus such as a computer or a mobile phone by wire or wirelessly, and transmit information of the angular velocity acquired by the angular velocity sensor 131 to the information processing apparatus. The information processing apparatus may then measure the pulse wave based on the angular velocity information. The information processing apparatus may execute, for example, a process of estimating glucose metabolism and lipid metabolism. In the case where the information processing apparatus connected to the electronic device 100 executes a variety of information processing, the electronic device 100 does not require the controller 143, the memory 145, the notification interface 147, etc. In the case where the electronic device 100 is connected to the information processing apparatus by wire, the electronic device 100 may not include the power source 144 and be supplied with power from the information processing apparatus.

The electronic device 100 may not include all movable portions described in the foregoing embodiments. The electronic device 100 may include only part of the movable portions described in the foregoing embodiments. For example, the measurement portion 120 may not be vertically displaceable with respect to the exterior portion 110. For example, the measurement portion 120 may not be rotatable with respect to the exterior portion 110.

Although the foregoing embodiments describe the case where the end 122a functions as a stopper that limits the displaceable range of the back portion 113, the part that functions as a stopper in the present disclosure is not limited to the end 122a. For example, the arm 133 may be provided with a stopper 200, as illustrated in FIG. 16. The stopper 200 may be located below the pulse pad 132 of the arm 133. In this case, the stopper 200 moves with vertical movement of the measurement portion 120, and therefore can function as a stopper even when the wrist of the subject is thin.

The electronic device 100 according to the foregoing embodiments may further include a wearing portion 160. FIG. 17 is a schematic external perspective diagram of a modification of the electronic device 100 in the case where the electronic device 100 includes the wearing portion 160. The wearing portion 160 may be, for example, attached to the cover 111 of the electronic device 100, as illustrated in FIG. 17.

The wearing portion 160 is a mechanism used to maintain the contact state between the measured part and the measurement portion 120. In the example illustrated in FIG. 17, the wearing portion 160 is a long and narrow strip-like band. In the example illustrated in FIG. 17, the wearing portion 160 is attached to the cover 111 so that one end 160a will be joined to the measurement portion 120 and the other end 160b will be located on the y-axis negative side.

In the case where the electronic device 100 includes the wearing portion 160, for example, in a state in which the measured part is in contact with the measurement portion 120, the subject fixes the other end 160b to the upper end of the back portion 113 so as to wrap the wearing portion 160 around the wrist. The other end 160b may be fixed to the upper end of the back portion 113 by any method. For example, the other end 160b may be fixed to the upper end of the back portion 113 by a hook. For example, the other end 160b may be fixed to the upper end of the back portion 113 by any other method such as a hook-and-loop fastener. Thus, in the case where the electronic device 100 includes the wearing portion 160, the contact state between the measured part and the measurement portion 120 can be stabilized by fixing the wrist to the electronic device 100 using the wearing portion 160. Since the positional relationship between the measured part and the measurement portion 120 is unlikely to change during measurement, the pulse wave can be measured stably, and accordingly the measurement accuracy is improved.

The electronic device 100 according to the foregoing embodiments may not include all of the components described above, as long as its biological information measurement function is not compromised. For example, the electronic device 100 may not include the cover 111 of the exterior portion 110.

In the electronic device 100, the cover 111 may be removably provided in the exterior portion 110. In detail, the cover 111 may be removable from the exterior portion 110, as illustrated in FIG. 18 as an example. In the case where the cover 111 is removable, the subject may remove the cover 111 when measuring the biological information. For example, in the case where the electronic device 100 is used without being placed on a stand, by removing the cover 111, the subject can easily hold the electronic device 100 when measuring the biological information.

The electronic device 100 in a state in which the cover 111 has been removed may be, for example, mounted on a base. FIG. 19 is an external perspective diagram illustrating an example of a state in which the electronic device 100 is mounted on a base 170. The base 170 may be a flat plate-shaped member. In the example illustrated in FIG. 19, the base 170 has a substantially rectangular shape as viewed from above.

FIG. 20 is a diagram illustrating an example of a state in which the electronic device 100 and the base 170 are joined together. FIG. 20 illustrates how the electronic device 100 and the base 170 are joined together, with part of the left side portion 112b of the electronic device 100 being cut away. As illustrated in FIG. 20, the base 170 has a projection 171 in a mounting location of the electronic device 100. The projection 171 extends in the x-axis direction. The electronic device 100 has a recess corresponding to the projection 171, in the bottom portion 114. The subject can mount the electronic device 100 on the base 170 by fitting the recess of the bottom portion 114 onto the projection 171 of the base 170. With such a configuration, the subject can easily determine the mounting position of the electronic device 100 on the base 170. The electronic device 100 and the base 170 need not necessarily be joined by the configuration illustrated in FIG. 20. The electronic device 100 and the base 170 may be attached to each other by any other method.

The base 170 may include a placement portion 172 at a different position from the mounting position of the electronic device 100. For example, the placement portion 172 is provided at a position where the subject places the wrist on the base 170 when measuring the biological information using the electronic device 100. The placement portion 172 may be, for example, made of a cushioning material.

The base 170 may include a wearing portion 173 on its upper surface. In the example illustrated in FIG. 19, the wearing portion 173 is a long and narrow strip-like band, as with the wearing portion 160 in FIG. 17. For example, in a state in which the measured part is in contact with the measurement portion 120 and the wrist is placed on the placement portion 172, the subject fixes an end 173b of the wearing portion 173 to the upper end of the back portion 113 so as to wrap the wearing portion 173 around the wrist. Thus, the wrist can be fixed to the electronic device 100.

In the case of measuring the biological information in a state in which the electronic device 100 is mounted on the base 170, the electronic device 100 can be stably placed on a stand such as a desk through the base 170. This contributes to a more stable contact state between the measured part and the measurement portion 120. Hence, by measuring the biological information in a state in which the electronic device 100 is mounted on the base 170, the pulse wave can be measured more stably, and the measurement accuracy can be improved.

### REFERENCE SIGNS LIST

- 100: electronic device
- 110: exterior portion
- 111: cover
- 111a, 133a, 160a: one end
- 112a, 112b: side portion
- 113: back portion
- 114: bottom portion
- 115: shaft
- 116a, 123: connection
- 120: measurement portion
- 121: body
- 122: wall portion
- 122a, 173b: end
- 123a: bearing
- 124: plate spring
- 130: sensor
- 131: angular velocity sensor
- 132: pulse pad
- 133: arm
- 133b, 160b: other end
- 140: elastic body
- 143: controller
- 144: power source
- 145: memory
- 146: communication interface
- 147: notification interface
- 150: mobile terminal
- 151: server
- 160, 173: wearing portion
- 170: base
- 171: projection
- 172: placement portion
- 200: stopper

## Claims

1. An electronic device comprising:
an exterior portion including a holding surface configured to be held by a user; and
a measurement portion displaceable with respect to the exterior portion,
wherein the measurement portion includes:
an arm displaceable according to a pulse wave of the user; and
a sensor configured to detect displacement of the arm based on the pulse wave.

2. The electronic device according to claim 1, wherein the measurement portion is displaceable along a displacement plane intersecting with the holding surface, and
the arm is displaceable in a direction intersecting with the displacement plane, according to the pulse wave of the user.

3. The electronic device according to claim 1, wherein the arm is displaceable in a direction along the holding surface.

4. The electronic device according to claim 2, wherein the exterior portion includes a shaft extending in a direction along the holding surface, and
the measurement portion is rotationally displaceable about the shaft as an axis.

5. The electronic device according to claim 1, wherein the exterior portion includes a cover displaceable in a direction substantially parallel to a displacement direction of the arm.

6. The electronic device according to claim 5, wherein the cover is removable from the exterior portion.

7. The electronic device according to claim 1, further comprising
a notification interface configured to notify a measurement result of biological information measured based on the displacement of the arm.

8. The electronic device according to claim 1, wherein the arm includes a pulse pad configured to be in contact with a measured part of the user.

9. The electronic device according to claim 1, further comprising
an elastic body configured to, in a state in which the measurement portion is in contact with a measured part, bias the arm toward the measured part.

10. The electronic device according to claim 1, wherein the sensor is configured to detect an angular change of the arm according to the pulse wave of the user.

11. The electronic device according to claim 1, wherein a natural frequency of the arm is substantially equal to a frequency of the pulse wave of the user.

12. The electronic device according to claim 1, wherein a natural frequency of the arm is 0.5 Hz or more and 2 Hz or less.

13. The electronic device according to claim 1, further comprising
a controller configured to calculate an index based on the pulse wave acquired by the sensor as a result of detecting the displacement of the arm,
wherein the controller is configured to estimate a state of glucose metabolism or lipid metabolism of the user from the calculated index.

14. The electronic device according to claim 13, wherein the controller is configured to calculate an index related to a reflected wave from the pulse wave acquired by the sensor, and estimate the state of glucose metabolism or lipid metabolism of the user from the calculated index related to the reflected wave.

15. The electronic device according to claim 1, further comprising
a controller configured to calculate an index based on the pulse wave acquired by the sensor as a result of detecting the displacement of the arm,
wherein the controller is configured to estimate blood fluidity of the user from the calculated index.

16. The electronic device according to claim 15, wherein the controller is configured to calculate an index related to a reflected wave from the pulse wave acquired by the sensor, and estimate the blood fluidity of the user from the calculated index related to the reflected wave.
